# EUROPEAN PATENT SPECIFICATION

(11) **EP 1 860 961 B1**
(45) Date of publication and mention of the grant of the patent: **05.05.2010**
(21) Application number: 06726491.1
(22) Date of filing: 23.03.2006
(51) Int. Cl.: A24C 5/34, G01N 27/49

(54) **SMOKING MACHINE**
RAUCHMASCHINE
MACHINE À FUMER

(30) Priority: 24.03.2005 GB 0506120
(43) Date of publication of application: 05.12.2007
(73) Proprietor: Molins PLC, Linford Wood East, Milton Keynes, Bucks, MK14 6LY (GB)
(72) Inventor: TINDALL, Ian Francis, Moredown Bournemouth BH9 2UJ (GB)
(74) Representative: Williams, Michael Ian
(86) International application number: PCT/GB2006/001073
(87) International publication number: WO 2006/100493

(56) References cited:
- EP-A- 0 419 143
- EP-A- 0 880 020
- DE-A1- 4 132 178
- DE-C1- 4 341 814
- US-A- 2 851 411
- US-A- 3 878 080
- US-A1- 2001 041 366
- US-B1- 6 305 214

## Description

The present invention relates to a smoking machine for smoking a smoking article, such as a cigarette or cigar, in order to analyse the composition of a smoke stream from the smoking article.

It is well known that the process of smoking cigarettes and cigars liberates many chemicals in the form of gases, vapours or particulates that are either harmful or noxious in character. These chemicals are formed as the result of the pyrolysis or partial pyrolysis of the article being smoked. The amount liberated by a particular cigarette is dependant upon the method of smoking, the tobacco blend, the packing of the tobacco, the permeability of the paper used in construction and any filter system, amongst other factors. Because there are so many variables involved in the process of producing smoke it is necessary to analyse actual smoke streams to determine the delivery of these gases and vapours.

Analysis of the composition of the smoke stream is important for two reasons. Firstly, the manufacturer of smoking articles may use smoke composition as a measure of the acceptability of the product, or as a quality control measure. Secondly, routine analysis of smoking articles is required by legislation for reporting to regulatory bodies to ensure the products sold to the consumer do not exceed maximum levels of particular hazardous substances.

The normal method employed for the analysis of smoke composition involves the use of a mechanical smoking machine with well defined smoking characteristics. The use of such a machine fixes a set of process variables concerned with smoke generation, so the variables affecting the formation of gases and vapours is then restricted to the design, construction and composition of the smoking article. There are two types of smoke formed, main stream which is formed when air is passed through the cigarette as a breath is drawn, and side stream, where the cigarette burns between puffs. The two smoke streams are likely to differ in composition as the temperature of pyrolysis will differ between the two modes.

The smoke so formed is normally captured in a fibre pad which is then removed for analysis. The removed capture pad requires a series of chemical extractions and analysis that may take many hours, considerable labour and sophisticated analytical instruments. Gases that are not captured by the pad may be bubbled through impingers to trap the gas chemically or physically, or the gases may be measured using sophisticated analytical instruments dedicated for the purpose.

The known method of analysing a smoke stream is characterised by the complexity of capture and analysis and by the time delay between smoking and analysing the captured gases. There is also a potential source of bias and error as the vapour or gas may be imperfectly captured or may be involved in a chemical reaction affected by thermodynamic factors. Whilst this is generally acceptable for trace elements, routine analysis for major components may be hampered by this slow process.

United States Patent Application Publication number US 2001/0041366 discloses a device for detecting the presence of an analyte, comprising a sample chamber having a fluid inlet port for the influx of the analyte, a fluid concentrator, and an array of sensors.

DE 4341814 discloses a technique for determining the residual mass of a cigarette which has been smoked by a smoking machine.

According to an aspect of the present invention there is provided a smoking machine for smoking a smoking article, the smoking machine comprising an electrochemical cell for analysing the composition of a smoke stream from the smoking article.

By using an electrochemical cell (sometimes known as a fuel cell) in a smoking machine, the present invention may provide the advantage that a gas or vapour phase chemical can be analysed directly in real time without the need for pre-conditioning or preparation.

The electrochemical cell is preferably arranged to detect a gas in the smoke stream. For example, the electrochemical cell may be arranged to oxidize or to reduce the gas to be detected. The electrochemical cell may be arranged to output an electrical signal indicative of the presence or amount of the gas to be detected. The smoking machine may further comprise an amplifier for amplifying the output of the electrochemical cell.

In order to carry out an analysis of the smoke stream, the smoking machine may further comprise processing means for processing the output of the electrochemical cell. The processing means may be arranged to log an amount of a detected gas over time as the smoking article is smoked, in order to build up a profile of the concentration of the gas through the smoking run. The processing means may be a processor co-located with the electrochemical cell, or it may be separate from the electrochemical cell. For example the processing means may be a separate computer which is arranged to log, analyse and display the data.

The concentration of a particular gas and the manner in which the concentration changes with time through a smoking run will, within predefined limits, be characteristic of the cigarette brand smoked. Thus a characteristic profile of the smoke generated for that brand may be developed for a standard set of mechanical smoking parameters. By comparing the characteristic profile with the profile of a cigarette under test, quality control or counterfeit detection may be performed on the test cigarette. Therefore the processing means may be arranged to store a profile of an amount of gas over time for a known smoking article and to compare the logged profile with the stored profile.

The electrochemical cell may be located in a chamber which is in communication with the smoke stream. The chamber may be a dedicated analysis chamber located in the path of the smoke stream. Such an arrangement may allow a conventional smoking machine to be modified simply by inserting the analysis chamber into the path of the smoke stream. Alternatively the chamber may be a filter pad chamber. This may allow the chamber to perform the dual functions of holding a filter pad and holding the electrochemical cell, which may allow the machine to be more compact and cheaper to manufacture.

The chamber may comprise a sub-chamber for accommodating the electrochemical cell. The sub-chamber may comprise a passageway which is selected in size to control the flow rate through the sub-chamber. Alternatively or in addition the smoking machine may further comprise means for introducing an additional gas, such as air, into the sub-chamber so as to dilute the smoke stream impinging on the electrochemical cell. These measures may help to ensure that the correct concentration of the gas to be detected impinges on the electrochemical cell.

Preferably the smoking machine comprises a plurality of electrochemical cells. Two or more electrochemical cells may in some cases be the same or similar. However two or more electrochemical cells are preferably sensitive to different gases and/or have a different sensitivity to a particular gas. This can allow the cells to provide simultaneous information about more than one substance in the smoke stream.

Each of the electrochemical cells may be located in a separate sub-chamber. Two or more sub-chambers may then comprise passageways of different sizes to control the flow rates through the sub-chambers. This may help to compensate for different sensitivities of the different electrochemical cells. Furthermore, two or more sub-chambers may be arranged to dilute the smoke stream by a different amount, which again may help to compensate for different sensitivities.

As the detection of a gas by an electrochemical cell may not be perfectly specific, the combination of a number of complimentary cells that have cross interferences to other gases can be used to determine more accurately the concentration of a specified gas. Therefore the smoking machine may further comprise processing means for determining a cross interference between different gases based on the output of two or more electrochemical cells. For example, the processor may be arranged to solve a set of at least two simultaneous equations involving at least two measurements and at least two unknowns. The equations may be linear or non-linear. Any appropriate technique may be used to solve the equations, such as direct or iterative methods.

Two electrochemical cells which are both sensitive to the same gas may be located one on either side of a filter pad. This arrangement may provide information concerning the efficiency of the filter pad. In this case processing means may be arranged to compare a concentration of a gas detected before the filter pad with a concentration of the gas detected after the filter pad.

The smoking machine may further comprise a gas permeable membrane located between the smoke stream and the electrochemical cell. Such a membrane may allow the gas of interest to pass through, but block the passage of particulates, condensates and unwanted solid and liquid materials. The membrane may be removable. The membrane may then perform the function of a particulate and condensate collection pad removing all particulates and condensates from the smoke stream. These collected materials may be extracted from the membrane for future analysis. Where a plurality of electrochemical cells are provided, some cells may be provided with a membrane and some without, or different cells may be provided with different membranes, in order to ensure that the gases of interest impinge on the electrochemical cells in the desired concentrations.

The smoking machine of the present invention may be arranged to analyse main stream smoke, or side stream smoke, or both.

Corresponding methods may also be provided. Thus, according to another aspect of the invention there is provided a method of analysing the composition of a smoke stream from a smoking article, the method comprising detecting the presence and/or quantity of a gas using an electrochemical cell.

Features of one aspect of the invention may be applied to any other aspect of the invention. Any of the apparatus features may be provided as method features, and *vice versa.*

It will be appreciated from the above that, under predefined mechanical smoking conditions the pattern of detection, analysis and response can be used to assess the characteristic build and composition of the smoking article and thus can be used to identify the brand, construction method or composition. The integrated concentration analysis of the smoke stream can be extrapolated mathematically to determine the total compositional delivery of one or more component of the whole smoking article.

Preferred features of the present invention will now be described, purely by way of example, with reference to the accompanying drawings, in which:
Figure 1 is a plan view of parts of a smoking machine in a first embodiment of the invention;
Figure 2 is a cross section through part of the machine of Figure 1;
Figure 3 is a conceptual sketch showing the principle of how the properties of a cigarette may be used to "fingerprint" brands;
Figure 4 is a plan view of parts of a smoking machine in a second embodiment of the invention;
Figure 5 shows parts of a smoking machine in a third embodiment of the invention;
Figure 6 is a block diagram of a processing component in an embodiment of the invention designed to calculate the concentrations of gases; and
Figure 7 is a block diagram of a processing component in an embodiment of the invention designed to match samples to known brands of cigarettes;

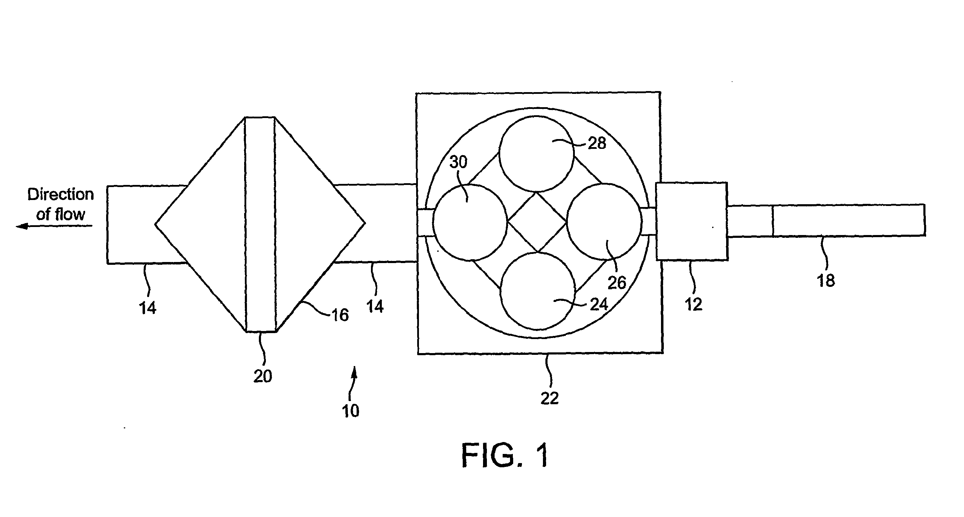

Referring to Figure 1, smoking machine 10 comprises cigarette holder 12, flow chamber 14, and filter pad chamber 16. A cigarette 18 is inserted into holder 12, and a filter pad 20 is inserted in filter pad chamber 16. In operation, the cigarette is lit and air is drawn through the cigarette at regular intervals, of regular duration and of consistent volume using a mechanically driven gas syringe or pump (not shown).

The smoking machine 10 also comprises an analysis chamber 22 which is located in the path of the main stream smoke between the smoked cigarette and the collection pad. The analysis chamber 22 is so designed that the volume is minimised to reduce the so called "dead volume" of the system. The analysis chamber 22 comprises four electrochemical cells 24, 26, 28, 30, each of which is fitted to the chamber in its own small well or sub-chamber.

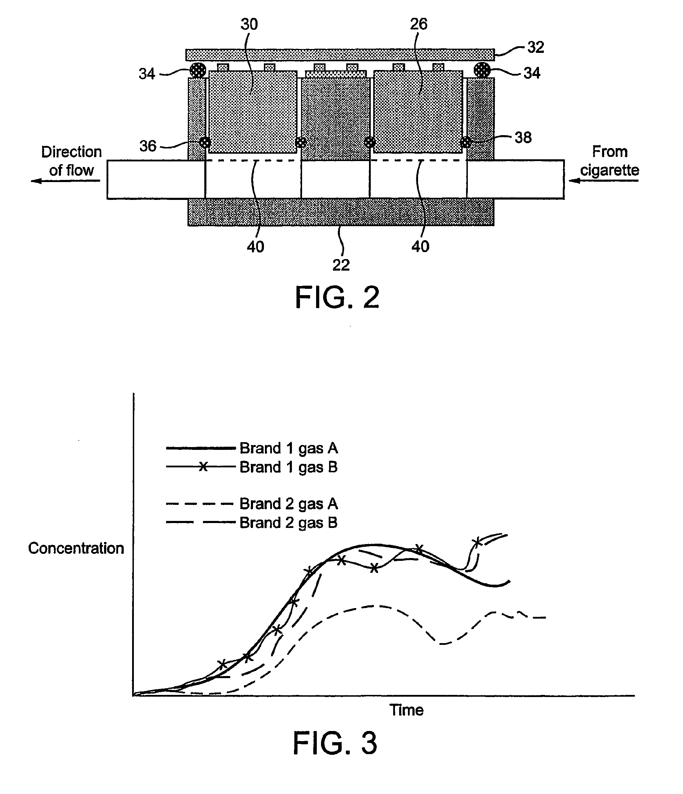

Figure 2 is a cross section through the analysis chamber 22. In Figure 2, two of the electrochemical cells 26, 30 are shown. The cells 26, 30 are sealed in place by means of elastomeric seals or o-rings 36, 38. These may be fitted either to the face of the cells or about their circumference, which ever is the most convenient. The purpose of these seals is to ensure that gases and vapours do not leak past the cells and cause dilution effects.

Each cell 26, 30 is fitted, normally by means of a plug and socket arrangement, to a printed circuit board (PCB) 32. The PCB 32 has resident electronics suitable for control of the cells, amplification of the currents produced, measurement of the currents and reporting the currents generated. It may be advantageous to include on this printed circuit board one or more microprocessors that convert this raw information into high level information concerning concentrations and composition. The PCB 32 may be connected to an external computer for analysing and displaying the information. The PCB 32 is also fitted with a seal 34 to prevent any minor leakage of gas from the system.

In front of the cells 26, 30 is a gas permeable membrane 40. The gas permeable membrane allows gases of interest to pass but prevents condensates and particulates, usually referred to as "tar" in the context of smoking, from passing. Again the volume between the membrane 40 and the cells 26, 30 is kept to a minimum to prevent a "dead volume" forming.

In operation, as the smoking article is smoked the smoke is drawn into the analysis chamber 22. The gases present begin to pass through the membrane 40 and enter the electrochemical fuel cells 24, 26, 28, 30. The cells have a small hole in the front of them which controls the diffusion of the gas through the gas permeable membrane. Gases which have passed through the membrane then encounter an electrode. This electrode catalyses a reaction with the gas either reducing or oxidising the gas. This liberates electrons which pass through the electrolyte in the cell to a second electrode where a complimentary reaction occurs. In practice none of the cell is consumed in the process and current flows in the cell proportional to the concentration of gas in the surrounding atmosphere.

The nature of the electrochemical cells is such that they are quasi specific to particular gases and so the presence of a current not only determines that a target gas is present but also its concentration. The reaction is such that very little of the gas under analysis reacts and is consumed and so can be considered to be a non consuming technology.

In practice it may be necessary to calibrate the individual sensor to the target gas to determine the response and provide a curve of concentration against current flowing.

As there is a mixture of gases formed in the smoking process, the relative concentrations of gases should be indicative of the brand being smoked. Moreover as the gas concentrations change as a smoking article is consumed (clogged filter, tobacco column acting as a filter will get smaller, less air entering smoke stream through the wrapping paper as this gets shorter, deliberate variations in tobacco packing density along rod and so on) this pattern through the smoking article will also be indicative of the brand.

By using an array of sensors as shown in Figures 1 and 2 additional benefits are achieved. Where individual cells are not perfectly specific detectors of a target gas the array concept allows compensation of measurement in one cell by one or more of the other cells. For example cell A detects and measures hydrogen cyanide (HCN), and cell B detects and measures carbon monoxide but there is also a slight effect from HCN. The cell A can determine the concentration of HCN and then the supervisory system can compensate for the effect of this HCN on cell B and so more perfectly calculate the residual effect of the carbon monoxide giving greater accuracy of measurement.

If desired, the analysis chamber 22 could be located after the filter pad chamber 16, or an analysis chamber could be located on either side of the filter pad chamber 16. In the later case one or more of the cells may be duplicated on the other side of the filter pad. This arrangement can not only provide information about the smoke stream composition but also information concerning the efficiency of the pad collection device.

Figure 3 is a conceptual sketch showing the principle of how the properties of the cigarette may be used to "fingerprint" brands. One gas type (B) has the same total concentration for the two brands but slightly dissimilar patterns of evolution. The other gas (A), measured simultaneously with gas B, has totally different total concentration and pattern of evolution. By looking at several elements for both total integrated concentration and the concentration in the time domain a unique picture of the brand can be formed and so become a "standard" for the brand. This can then be used to assess manufacturing consistency. For example, it may be determined whether a latest batch matches the profile for the brand. Alternatively this technique can be used in the detection of counterfeit products. If the smoked profile does not match the known profile of a particular brand then the cigarette is likely to be counterfeit.

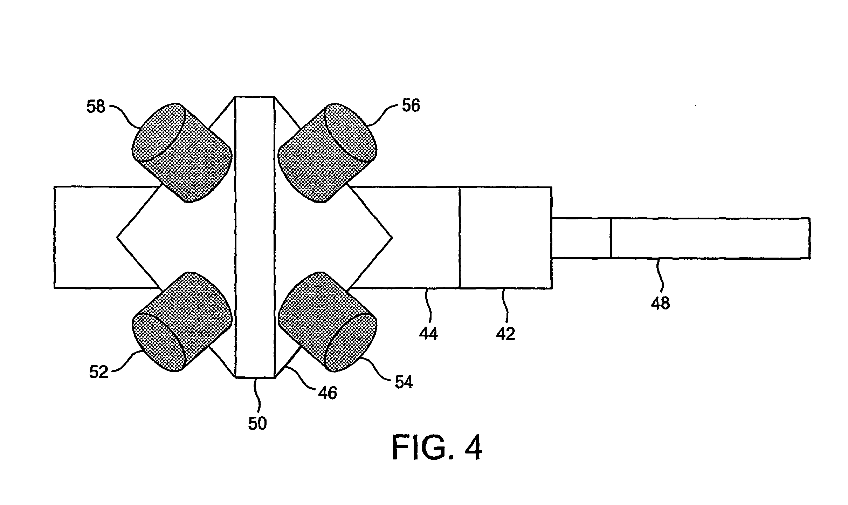

Figure 4 is a plan view of parts of a smoking machine in a second embodiment of the invention. In this embodiment the existing chamber used for collection of the mainstream gases on a filter pad is modified to allow direct insertion of the electrochemical or fuel cells.

Referring to Figure 4, the smoking machine comprises cigarette holder 42, flow chamber 44, and filter pad chamber 46. A cigarette 48 is inserted into holder 42, and a filter pad 50 is inserted in filter pad chamber 46. A mechanically driven pump or gas syringe (not shown) is used to draw air through the cigarette 48, flow chamber 44, and filter pad chamber 46.

As can be seen in Figure 4, electrochemical cells 52, 54, 56, 58 are fitted to the filter pad chamber 46. In this embodiment cells 54, 56 are located before the filter pad and cells 52, 58 are located after the filter pad. The cells 52, 54, 56, 58 are sealed in place by means of elastomeric seals or o-rings (not shown). The seals may be fitted either to the face of the cells or about the circumference, which ever is the most convenient. The purpose of the seals is to ensure that gases and vapours do not leak past the cell and cause dilution effects.

Each cell 52, 54, 56, 58 is fitted by means of a plug and socket arrangement to a flexible printed circuit board. The printed circuit board has resident electronics suitable for control of the cells, amplification of the currents produced, measurement of the currents and reporting the currents generated. It may be advantageous to include on this printed circuit board one or more microprocessors that convert this raw information into high level information concerning concentrations and composition. The PCB may be connected to an external computer for analysing and displaying the information.

Where the cells are between the smoking article and pad it may be necessary to attach a gas permeable membrane that allows the gases of interest to pass but prevents condensates and particulates, usually referred to as "tar" in the context of smoking, from passing. Again the volume between the membrane and the cell will be kept to a minimum to prevent a "dead volume" forming. Those cells between the pad and the gas syringe may not need such a membrane as the filter pad forms this function.

As the smoking article is smoked the smoke is drawn into the filter pad chamber 46. The gases present begin to pass through the membrane and enter the electrochemical fuel cells 52, 54, 56, 58 where a current is generated, amplified and measured.

By using an array of sensors as shown in Figure 4 the same additional benefits as described above with reference to the first embodiment can be achieved. The measurements can determine the total concentration of compound liberated under a predetermined set of conditions and also the "fingerprint" for the brand under those same conditions.

In this embodiment the electrochemical cells can be either before or after the filter pad. If required, one or more of the cells may be duplicated on the other side of the filter pad. This arrangement can not only provide information about the smoke stream composition but also information concerning the efficiency of the pad collection device.

If desired, electrochemical cells may be located in a separate analysis chamber (as in the first embodiment) as well as in the filter pad chamber 46.

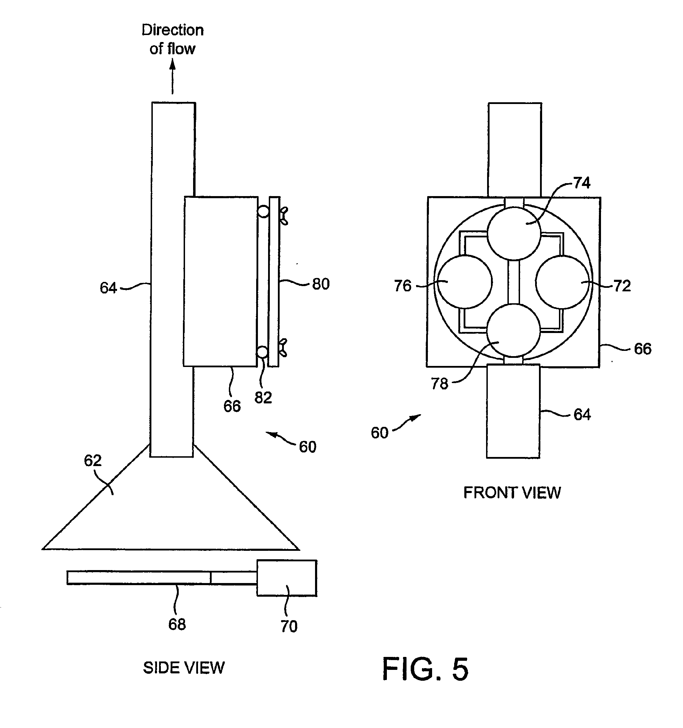

Figure 5 shows parts of a smoking machine in a third embodiment of the invention. In this embodiment a dedicated chamber for analysing a side smoke stream is fitted to a chimney above the smoking article. The side stream smoke is again characteristic of the brand and the mechanical smoking regime used to generate the main stream smoke.

Referring to Figure 5, side stream smoking machine 60 comprises collection hood 62, chimney 64 and gas analysis chamber 66. The collection hood 62 is located above cigarette 68 which is held by cigarette holder 70. The cigarette holder 70 is connected to a main stream smoking machine (not shown), such as a smoking machine in the first or second embodiment described above, in order to smoke the cigarette in a predetermined manner. Side stream smoking machine 60 also comprises a pump or mechanically driven gas syringe (not shown) which draws smoke from the cigarette 68 through the collection hood 62 and chimney 64 when the main stream smoking machine is not puffing.

The gas analysis chamber 66 comprises a number of sub-chambers, each of which houses a respective electrochemical cell 72, 74, 76, 78. The cells are sealed in place by means of an elastomeric seal or o-ring (not shown). Each cell is fitted, normally by means of a plug and socket arrangement, to a printed circuit board 80. The printed circuit board 80 has resident electronics suitable for control of the cells, amplification of the currents produced, measurement of the currents and reporting the currents generated. The printed circuit board may include one or more microprocessors that convert this raw information into high level information concerning concentrations and composition, and/or it may be connected to an external computer for analysing and displaying the information. The PCB 80 is also fitted with a seal 82 to prevent any minor leakage of gas from the system.

In front of the cells is a gas permeable membrane that allows the gases of interest to pass but prevents condensates and particulates from passing.

In between puffs from the mechanical smoking machine the smoke formed from the smouldering smoking article is drawn past the analysis chamber 66. Some of this gas will be drawn into the chamber and detected by the electrochemical cells 72, 74, 76, 78. With knowledge of the flow rate though the chimney 64, and hence the dilution, the amount of gas detected can be related to the total amount of the gas generated in the side stream process.

By using an array of electrochemical cells as shown in Figure 5 the same additional benefits as described above with reference to the first and second embodiments may be achieved.

If the side stream smoking machine includes a filter pad chamber, then the electrochemical cells may be located in the filter pad chamber instead of or in addition to in the analysis chamber.

The side stream smoking machine of the third embodiment may be used in conjunction with the main stream smoking machine of the first or second embodiments.

The embodiments described above allow specific gas and vapour concentrations in mainstream and side stream smoke to be determined quickly and simply for mechanically smoked cigarettes and cigars. The embodiments are based upon the catalytic oxidation or reduction of the gas or vapour to be measured within an electrochemical cell. The combination of the mechanical smoking and simple multigas measurement capability of electrochemical cells provides a powerful analytical tool that can not only analyse gas constituents but under well defined standard smoking conditions provide a distinctive pattern of response and concentration that could be considered a "fingerprint" characteristic of the brand of cigarette smoked.

Further details of various analysis techniques used in embodiments of the invention are discussed below.

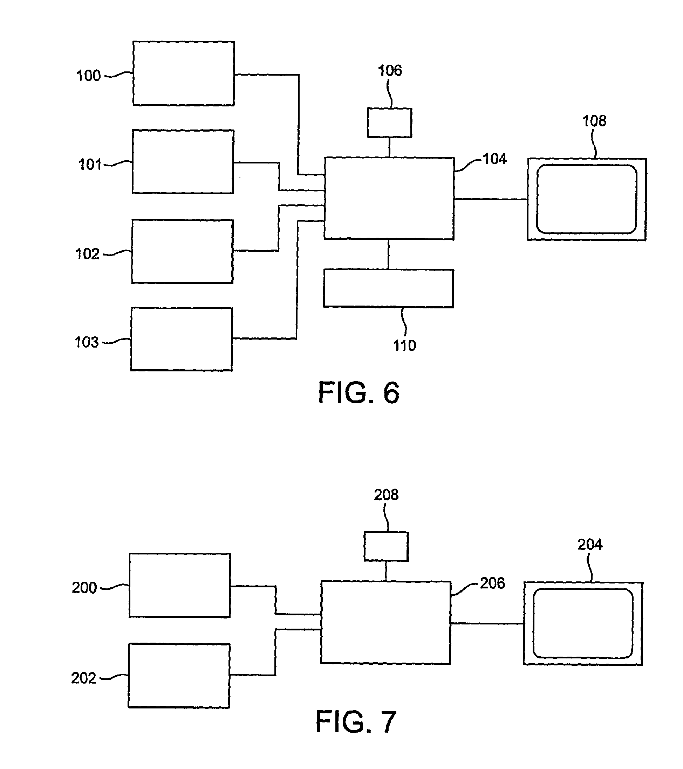

Figure 6 shows four electrochemical cells 100, 101, 102, 103 arranged to provide outputs to a processor 104. Processor 104 may be part of a computer, or alternatively may be integrated within a smoking machine. The processor 104 is arranged for electrical connection with: data storage means 106, which may be a memory chip; a display unit 108; and a keyboard 110, which is a data input device.

The magnitudes of the outputs of each of sensors 100, 101, 102, and 103 may be (approximately) linearly dependent on the concentrations of four gases. Thus, provided that the output of each sensor is dependent on a unique combination of the four gases, then a unique solution may be found for the true concentration of each gas. Mathematically speaking, this corresponds to a situation where there are four independent measurements and four unknowns; this is classically soluble by matrix algebra. Where there are "N" unknowns, the solution of the system of equations involves the inversion of an NxN matrix. Such a calculation can be performed by a suitably programmed processor, as will be apparent to one skilled in the art.

In many real-life situations the outputs of sensors 100, 101, 102 and 103 will be non-linear. This non-linearity may be dependent on various factors such as output magnitude or a specific gas concentration. If the nature of non-linear relationship can be determined, then the system may be calibrated to compensate for this (so long as the non-linearity is a function of a known parameter). If any non-linear effects can be accounted for then the system will once again be easily soluble through matrix manipulation.

Processor 104 is arranged for electrical connection to memory 106. Processor 104 may read data from memory 106, which data may be related to any stored non-linear relationships. Processor 104 may also write to memory 106 since it may be desirable to store gas concentrations at any point in time, or to store a log of gas concentrations against time. Keyboard 110 is arranged for electrical connection to the processor 104 and may be used to input data to the processor, which data may then be stored in memory 106. Keyboard 110 may be used to update any data used by the processor 104 or to amend manually any recorded data. Display 108 is also arranged for electrical connection to processor 104, and may be arranged to display the concentration of any gas at any instant in time. Display 108 may also display a real-time log of the concentrations of different gases as they change in time.

Although four sensors are depicted in Figure 6 it will be apparent that any number could be used.

Figure 7 shows parts of a "fingerprinting" apparatus. The apparatus comprises electrochemical cells 200 and 202 in electrical connection with a processor 206. Processor 206 is further arranged for electrical connection to a display 204, and memory 208.

Sensors 200 and 202 are both sensitive to some combination of two gases. This dependency may be linear or non-linear, but each sensor should be dependent on a unique combination of two gases so that a unique solution may be found for the concentration of each gas. In either case, processor 206 calculates the concentration of each gas.

Processor 206 may be arranged to operate as a data logger - i.e. the processor may sample data regularly over a period of time. Any logged data may be stored in memory 208 and/or displayed on display 204.

Either during or after data logging, the processor carries out "fingerprinting", to determine the brand of cigarette that produces a particular pattern of gas concentration evolution. This type of matching may be achieved by many techniques and by way of example an exemplary technique is described below.

Memory 208 comprises patterns of known gas concentration evolutions for a plurality of gases and a plurality of types of cigarette. Processor 206 samples the concentration of each of the two gases and then compares this figure with each of the stored profiles to yield a deviation for each. In this way a deviation may be determined for each sample at an instant in time with respect to each stored profile. By finding a deviation for each sampled point over the length of the stored profile and averaging, a standard deviation for the evolution of each gas may be found with respect to each stored profile. The stored profile with the lowest associated standard deviation is then the most likely candidate to match with the sample. However, having the lowest associated standard deviation does not necessarily mean a match between a candidate stored profile and the sample. Memory 208 stores a maximum standard deviation threshold, and if the lowest standard deviation is above this threshold then processor 206 will not find a match.

This technique provides a method for matching smoke evolution profiles from sample cigarettes to particular brands. Particular brands may be thus identified, and cigarettes not corresponding to known brands which may be counterfeits may also be identified.

If a counterfeit cigarette is found then this may be stored in the memory and/or displayed on display 204.

Rather than identifying individual sample cigarettes as counterfeits, the processor 206 may be arranged to display the standard deviation of the sample with respect to each stored profile on display 204. This may be done in combination with a "confidence factor" showing how likely a match is for the sample with each stored profile.

As mentioned, the processor 206 may carry out "fingerprinting" during the data logging process. The likelihood of obtaining a high confidence match increases with time in a calculable way. Therefore it may be desirable to display each possible match on display 204 together with an accuracy/certainty parameter which may be conveniently displayable as a percentage.

Although Figure 7 only shows two electrochemical cells, it will be appreciated that three or more could be used, to detect a greater number of gases. In addition, other "fingerprinting" techniques could be used. A possible alternative could include convolving logged data with stored profiles and searching for peak responses.

## Claims

1. A smoking machine (10) for smoking a smoking article (18), **characterised in that** the smoking machine comprises an electrochemical cell for analysing the composition of a smoke stream from the smoking article.

2. A smoking machine according to claim 1 wherein the electrochemical cell is arranged to detect a gas in the smoke stream.

3. A smoking machine according to claim 1 or 2 wherein the electrochemical cell is arranged to oxidize or to reduce the gas to be detected.

4. A smoking machine according to any of claims 1 to 3 wherein the electrochemical cell is arranged to output an electrical signal indicative of the presence or amount of the gas to be detected.

5. A smoking machine according to claim 4 further comprising an amplifier for amplifying the output of the electromagnetic cell.

6. A smoking machine according to any of the preceding claims further comprising processing means for processing the output of the electrochemical cell.

7. A smoking machine according to claim 6 wherein the processing means is arranged to log an amount of a detected gas over time as the smoking article is smoked.

8. A smoking machine according to claim 7 wherein the processing means is arranged to store a profile of an amount of a gas over time for a known smoking article and to compare the logged profile with the stored profile.

9. A smoking machine according to any of the preceding claims, wherein the electrochemical cell is located in a chamber which is in communication with the smoke stream.

10. A smoking machine according to claim 9 wherein the chamber is a dedicated analysis chamber located in the path of the smoke stream.

11. A smoking machine according to claim 9 wherein the chamber is a filter pad chamber.

12. A smoking machine according to any of claims 9 to 11 wherein the chamber comprises a sub-chamber for accommodating the electrochemical cell.

13. A smoking machine according to claim 12 wherein the sub-chamber comprises a passageway which is selected in size to control the flow rate through the sub-chamber.

14. A smoking machine according to claim 12 or 13 further comprising means for introducing an additional gas into the sub-chamber.

15. A smoking machine according to any of the preceding claims, comprising a plurality of electrochemical cells.

16. A smoking machine according to claim 15, wherein two or more electrochemical cells are sensitive to different gases.

17. A smoking machine according to claim 15 or 16, wherein each of the electrochemical cells is located in a separate sub-chamber.

18. A smoking machine according to claim 17, wherein two or more sub-chambers comprise passageways of different sizes to control the flow rates through the sub-chambers.

19. A smoking machine according to claim 17 or 18, wherein two or more sub-chambers are arranged to dilute the smoke stream by a different amount.

20. A smoking machine according to any of claims 15 to 19 further comprising processing means for determining a cross interference between different gases based on the output of two or more electrochemical cells.

21. A smoking machine according to claim 20, wherein the processor is arranged to solve a set of at least two simultaneous equations involving at least two measurements and at least two unknowns.

22. A smoking machine according to any of claims 15 to 21 wherein two electrochemical cells which are both sensitive to the same gas are located one on either side of a filter pad.

23. A smoking machine according to claim 22 further comprising processing means for comparing a concentration of a gas detected before the filter pad with a concentration of the gas detected after the filter pad.

24. A smoking machine according to any of the preceding claims, further comprising a gas permeable membrane located between the smoking article and the electrochemical cell.

25. A smoking machine according to claim 24 wherein the membrane is removable.

26. A smoking machine according to any of the preceding claims, arranged to analyse main stream smoke.

27. A smoking machine according to any of the preceding claims, arranged to analyse side stream smoke.

28. A method of analysing the composition of a smoke stream from a smoking article, the method comprising smoking the smoking article, **characterised by** detecting the presence and/or quantity of a gas in the smoke stream using an electrochemical cell.

## Patentansprüche

1. Eine Rauchmaschine (10) zum Rauchen einer Rauchware (18), **dadurch gekennzeichnet, dass** die Rauchmaschine eine elektrochemische Zelle zum Analysieren der Zusammensetzung eines Rauchstroms von der Rauchware umfasst.

2. Eine Rauchmaschine gemäß Anspruch 1, während die elektrochemische Zelle angeordnet ist, um ein Gas in dem Rauchstrom zu erfassen.

3. Eine Rauchmaschine gemäß Anspruch 1 oder 2, während die elektrochemische Zelle angeordnet ist, um das zu erfassende Gas zu oxidieren oder zu reduzieren.

4. Eine Rauchmaschine gemäß einem der Ansprüche 1 bis 3, während die elektrochemische Zelle angeordnet ist, um ein elektrisches Signal auszugeben, das eine Anwesenheit oder eine Menge des zu erfassenden Gases anzeigt.

5. Eine Rauchmaschine gemäß Anspruch 4, weiterhin umfassend einen Verstärker zum Verstärken des Ausgangs der elektromagnetischen Zelle.

6. Eine Rauchmaschine gemäß einem der vorhergehenden Ansprüche, weiterhin umfassend Verarbeitungsmittel zum Verarbeiten des Ausgangs der elektrochemischen Zelle.

7. Eine Rauchmaschine gemäß Anspruch 6, während das Verarbeitungsmittel angeordnet ist, um eine Menge an erfasstem Gas über die Zeit aufzuzeichnen, wenn die Rauchware geraucht wird.

8. Eine Rauchmaschine gemäß Anspruch 7, während das Verarbeitungsmittel angeordnet ist, um ein Profil einer Menge an Gas über die Zeit für eine bekannte Rauchware zu speichern und das aufgezeichnete Profil mit dem gespeicherten Profil zu vergleichen.

9. Eine Rauchmaschine gemäß einem der vorhergehenden Ansprüche, während die elektrochemische Zelle in einer Kammer angeordnet ist, die mit dem Rauchstrom in Verbindung steht.

10. Eine Rauchmaschine gemäß Anspruch 9, während die Kammer eine dedizierte Analysenkammer ist, die in dem Weg des Rauchstroms angeordnet ist.

11. Eine Rauchmaschine gemäß Anspruch 9, während die Kammer eine Filtermattenkammer ist.

12. Eine Rauchmaschine gemäß einem der Ansprüche 9 bis 11, während die Kammer eine Unterkammer zum Aufnehmen der elektrochemischen Zelle aufweist.

13. Eine Rauchmaschine gemäß Anspruch 12, während die Unterkammer einen Durchgang umfasst, der in seiner Größe ausgewählt ist, um die Flussrate durch die Unterkammer zu steuern.

14. Eine Rauchmaschine gemäß Anspruch 12 oder 13, weiterhin umfassend Mittel zum Einführen eines zusätzlichen Gases in die Unterkammer.

15. Eine Rauchmaschine gemäß einem der vorhergehenden Ansprüche, umfassend eine Mehrzahl an elektrochemischen Zellen.

16. Eine Rauchmaschine gemäß Anspruch 15, während zwei oder mehr elektrochemische Zellen sensitiv auf verschiedene Gase sind.

17. Eine Rauchmaschine gemäß Anspruch 15 oder 16, während jede der elektrochemischen Zellen in einer getrennten Unterkammer angeordnet ist.

18. Eine Rauchmaschine gemäß Anspruch 17, während zwei oder mehr Unterkammern Durchgänge von verschiedener Größe umfassen, um die Flussraten durch die Unterkammern zu steuern.

19. Eine Rauchmaschine gemäß Anspruch 17 oder 18, während zwei oder mehr Unterkammern angeordnet sind, um den Rauchstrom durch eine verschiedene Menge zu verdünnen.

20. Eine Rauchmaschine gemäß einem der Ansprüche 15 bis 19, weiterhin umfassend Verarbeitungsmittel zum Bestimmen einer Querempfindlichkeit zwischen verschiedenen Gasen basierend auf der Ausgabe von zwei oder mehr elektrochemischen Zellen.

21. Eine Rauchmaschine gemäß Anspruch 20, während der Prozessor angeordnet ist, um einen Satz von mindestens zwei gleichzeitigen Gleichungen zu lösen, die mindestens zwei Messwerte und mindestens zwei Unbekannte aufweisen.

22. Eine Rauchmaschine gemäß einem der Ansprüche 15 bis 21, während von zwei elektrochemische Zellen, die beide sensitiv auf dasselbe Gas sind, eine auf jeder Seite einer Filtermatte angeordnet ist.

23. Eine Rauchmaschine gemäß Anspruch 22, weiterhin umfassend Verarbeitungsmittel zum Vergleichen einer Konzentration an Gas, die vor der Filtermatte erfasst wurde, mit einer Konzentration des Gases, das nach der Filtermatte erfasst wurde.

24. Eine Rauchmaschine gemäß einem der vorhergehenden Ansprüche, weiterhin umfassend eine gasdurchlässige Membran, die zwischen der Rauchware und der elektrochemischen Zelle angeordnet ist.

25. Eine Rauchmaschine gemäß Anspruch 24, während die Membran entfernbar ist.

26. Eine Rauchmaschine gemäß einem der vorhergehenden Ansprüche, die angeordnet ist, um einen Hauptrauchstrom zu analysieren.

27. Eine Rauchmaschine gemäß einem der vorhergehenden Ansprüche, die angeordnet ist, um einen Seitenrauchstrom zu analysieren.

28. Ein Verfahren des Analysierens der Zusammensetzung eines Rauchstroms von einer Rauchware, wobei das Verfahren umfasst: Rauchen der Rauchware, **gekennzeichnet durch** Erfassen der Anwesenheit und/oder Menge eines Gases in dem Rauchstrom unter Verwendung einer elektrochemischen Zelle.

## Revendications

1. Machine à fumer (10) pour fumer un article à fumer (18), **caractérisée en ce que** la machine à fumer comprend une cellule électrochimique pour analyser la composition d'un écoulement des fumées de l'article à fumer.

2. Machine à fumer selon la revendication 1, dans laquelle la cellule électrochimique est agencée pour détecter un gaz dans l'écoulement des fumées.

3. Machine à fumer selon la revendication 1 ou 2, dans laquelle la cellule électrochimique est agencée pour oxyder ou pour réduire le gaz à détecter.

4. Machine à fumer selon l'une quelconque des revendications 1 à 3, dans laquelle la cellule électrochimique est agencée pour émettre un signal électrique indicatif de la présence ou de la quantité de gaz à détecter.

5. Machine à fumer selon la revendication 4, comprenant en outre un amplificateur pour amplifier la sortie de la cellule électrochimique.

6. Machine à fumer selon l'une quelconque des revendications précédentes, comprenant en outre un moyen de traitement pour traiter la sortie de la cellule électrochimique.

7. Machine à fumer selon la revendication 6, dans laquelle le moyen de traitement est agencé pour enregistrer une quantité de gaz détectée pendant le temps durant lequel l'article à fumer est fumé.

8. Machine à fumer selon la revendication 7, dans laquelle le moyen de traitement est agencé pour stocker un profil d'une quantité de gaz durant un temps pour un article à fumer connu et à comparer le profil enregistré avec le profil stocké.

9. Machine à fumer selon l'une quelconque des revendications précédentes, dans laquelle la cellule électrochimique se situe dans une chambre qui est en communication avec l'écoulement des fumées.

10. Machine à fumer selon la revendication 9, dans laquelle la chambre est une chambre d'analyse dédiée située dans le chemin de l'écoulement des fumées.

11. Machine à fumer selon la revendication 9, dans laquelle la chambre est une chambre à coussinet filtrant.

12. Machine à fumer selon l'une quelconque des revendications 9 à 11, dans laquelle la chambre comprend une sous-chambre pour loger la cellule électrochimique.

13. Machine à fumer selon la revendication 12, dans laquelle la sous-chambre comprend un passage qui est sélectionné en taille pour commander le débit d'écoulement à travers la sous-chambre.

14. Machine à fumer selon la revendication 12 ou 13, comprenant en outre un moyen pour introduire un gaz additionnel dans la sous-chambre.

15. Machine à fumer selon l'une quelconque des revendications précédentes, comprenant une pluralité de cellules électrochimiques.

16. Machine à fumer selon la revendication 15, dans laquelle deux ou plusieurs cellules électrochimiques réagissent à des gaz différents.

17. Machine à fumer selon la revendication 15 ou 16, dans laquelle chacune des cellules électrochimique se situe dans une sous-chambre séparée.

18. Machine à fumer selon la revendication 17, dans laquelle deux ou plusieurs sous-chambres comprennent des passages de tailles différentes pour régler les débits d'écoulement à travers les sous-chambres.

19. Machine à fumer selon la revendication 17 ou 18, dans laquelle deux ou plusieurs sous-chambres sont agencées pour diluer l'écoulement des fumées par une quantité différente.

20. Machine à fumer selon l'une quelconque des revendications 15 à 19, comprenant en outre un moyen de traitement pour déterminer une interférence croisée entre différents gaz sur la base de la sortie de deux ou plusieurs cellules électrochimiques.

21. Machine à fumer selon la revendication 20, dans laquelle le processeur est agencé pour résoudre un ensemble d'au moins deux équations simultanées impliquant au moins deux mesures et au moins deux inconnues.

22. Machine à fumer selon l'une quelconque des revendications 15 à 21, dans laquelle deux cellules électrochimiques, qui réagissent toutes les deux au même gaz, sont situées, une de chaque côté, d'un coussinet filtrant.

23. Machine à fumer selon la revendication 22, comprenant en outre un moyen de traitement pour comparer une concentration d'un gaz détectée avant le coussinet filtrant avec une concentration de gaz détectée après le coussinet filtrant.

24. Machine à fumer selon l'une quelconque des revendications précédentes, comprenant en outre une membrane perméable aux gaz située entre l'article à fumer et la cellule électrochimique.

25. Machine à fumer selon la revendication 24, dans laquelle la membrane est retirable.

26. Machine à fumer selon l'une quelconque des revendications précédentes, agencée pour analyser un écoulement principal des fumées.

27. Machine à fumer selon l'une quelconque des revendications précédentes, agencée pour analyser un écoulement latéral des fumées.

28. Procédé d'analyse de la composition d'un écoulement de fumées d'un article à fumer, le procédé comprenant le fumage de l'article à fumer, **caractérisé en** détectant la présence et/ou la quantité d'un gaz dans l'écoulement des fumées en utilisant une cellule électrochimique.
